# FASCICULE DE BREVET EUROPEEN

(11) **EP 0 600 014 B1**
(45) Date de publication et mention de la délivrance du brevet: **23.04.1997**
(21) Numéro de dépôt: 92918975.1
(22) Date de dépôt: 18.08.1992
(51) Int. Cl.: G01S 5/16, A61B 6/12, A61B 17/22

(54) **PROCEDE ET APPAREIL DE DETERMINATION DE LA POSITION EXACTE D'UNE CIBLE A L'AIDE D'UN DISPOSITIF DE RECEPTION COMPRENANT UNE PARTIE ACTIVE LINEAIRE FORMEE D'UNE MULTIPLICITE D'ELEMENTS DISCRETS SENSIBLES AUX RAYONNEMENTS**
VERFAHREN UND VORRICHTUNG ZUR GENAUEN ORTUNG EINES ZIELES UNTER VERWENDUNG EINER EMPFANGSEINRICHTUNG, DIE EINE STRAHLSENSORENZEILE ENTHÄLT
PROCESS AND APPARATUS FOR DETERMINING THE EXACT POSITION OF A TARGET USING A RECEIVING DEVICE COMPRISING A LINEAR ACTIVE PART FORMED FROM A VARIETY OF DISCRETE RADIATION-SENSITIVE ELEMENTS

(30) Priorité: 21.08.1991 FR 9110498; 30.04.1992 FR 9205413
(43) Date de publication de la demande: 08.06.1994
(73) Titulaire: TECHNOMED MEDICAL SYSTEMS, 69120 Vaulx-en-Velin (FR)
(72) Inventeur: DANCER, Paul, F-42100 Saint-Etienne (FR)
(74) Mandataire: Portal, Gérard
(86) Numéro de dépôt international: FR9200798
(87) Numéro de publication internationale: WO9304380

(56) Documents cités:
- EP-A- 0 260 550
- EP-A- 0 318 106
- EP-A- 0 449 113
- DE-U- 8 713 524
- US-A- 4 041 315
- US-A- 4 764 944

## Description

La présente invention concerne essentiellement un procédé et un appareil de détermination de la position exacte d'une cible par rapport à un repère de référence de coordonnées connues à l'aide d'un dispositif de réception comprenant une partie active linéaire formée d'une multiplicité d'éléments discrets sensibles aux rayonnements.

L'invention concerne plus particulièrement aussi l'utilisation de ce procédé et de cet appareil de détermination de la position exacte d'une cible dans le cadre d'un appareil de traitement d'une cible, de préférence par ondes de pression. Selon un mode de réalisation préféré, il s'agit d'un appareil de traitement par ondes de pression d'une cible choisie parmi le groupe consistant d'une lithiase, par exemple une lithiase rénale ou biliaire ; de tissus, par exemple des tumeurs bénignes ou malignes ; des os, par exemple une fracture ou une zone osseuse à traiter, notamment une zone d'ostéoporose.

On connaît jusqu'à présent divers procédés et appareils pour déterminer la position exacte d'une cible, comprenant l'emploi d'une source de rayonnement émettant un rayonnement capable d'être reçu par un dispositif de réception de ce rayonnement, ladite source et ledit dispositif de réception étant disposés de part et d'autre de ladite cible (voir EP-A-O 240 565 ou EP-A-O 260 550).

Le document Siemens DE-U-87 13524 = US-A-4 914 588 décrit un appareil de formation d'image par tomographie nécessitant pour construire l'image une mise en rotation autour d'un axe de rotation parallèle au corps du patient pour tourner autour de celui-ci. Le patient est en général déplacé avec la table de support et ce dispositif nécessite un système ultrasonique de localisation complémentaire.

De même, le document EP-A-318 106 Philips = US-A-4 967 735 décrit un appareil de destruction de concrétion comprenant un ensemble fluoroscopique combiné à un ensemble de détection de photons diffusés, avec emploi d'un diaphragme comportant une position de pleine ouverture et une position d'ouverture réduite. La position notamment de la source de rayonnement apparaît être fixe ce qui conduit généralement à une irradiation importante du patient.

On connaît par ailleurs, par le document US-A-4 764 944 (Finlayson) un procédé et un dispositif pour positionner un calcul se trouvant à l'intérieur d'un rein d'un patient utilisant deux sources de rayonnement pénétrant convergent se croisant au point focal F2 constituant le foyer externe d'une ellipsoïde où sont générées des ondes de choc. Ici la position initiale des sources n'est pas quelconque, mais au contraire doit être bien précise pour se croiser au point focal. En outre, la solution technique proposée par Finlayson nécessite une étape de calibrage pour déterminer la valeur de l'angle a entre les coordonnées dans le plan d'observation et déterminer l'angle entre le plan d'observation et l'horizontale. On utilise en particulier une tige se terminant par une bille que l'on observe jusqu'à ce que la radiation observée par les fluoroscopes soit centrée sur le point d'intersection de chacun des écrans. Cette procédure est donc extrêmement compliquée et exige dans tous les cas l'utilisation de deux sources et de deux dispositifs de réception, en particulier des fluoroscopes dont la position initiale doit être bien déterminée pour chacun d'eux par son second foyer, ce qui exige que ces sources soient liées en permanence au dispositif de traitement, ici une ellipsoïde. En outre, un calibrage est nécessaire à l'aide du dispositif comportant la bille pour amener l'image de la bille au centre des écrans des fluoroscopes.

Par ailleurs, le déposant a également décrit dans le document WO-A-91/07913 un procédé et un appareil de détermination de la position d'une cible utilisant un dispositif de masquage combiné à un film sensible aux rayons X. Cette solution qui est sûre et fiable exige cependant un temps de développement minimum du film qui n'est pas toujours compatible avec les exigences industrielles et médicales.

La présente invention a donc pour but principal de résoudre le nouveau problème technique consistant en la fourniture d'une solution permettant de déterminer la position exacte d'une cible en un temps relativement court tout en étant d'une grande fiabilité et reproductibilité.

La présente invention a encore pour but principal de résoudre le nouveau problème technique consistant en la fourniture d'une solution permettant de déterminer la position exacte d'une cible sans avoir à réaliser un déplacement quelconque du dispositif de traitement du patient et/ou du patient.

La présente invention a encore pour but principal de résoudre le nouveau problème technique consistant en la fourniture d'un procédé et d'un appareil pour déterminer la position exacte d'une cible sans avoir à amener cette cible dans une position déterminée relativement au dispositif de traitement et/ou sans avoir à l'amener dans une position déterminée relativement au dispositif de réception du rayonnement.

La présente invention a encore pour but principal de résoudre le nouveau problème technique consistant en la fourniture d'un procédé et d'un appareil pour déterminer la position exacte d'une cible utilisant un rayonnement d'intensité sensiblement uniforme lors de sa réception par le dispositif de réception, afin d'améliorer la qualité de l'image obtenue.

La présente invention a encore pour but de résoudre le nouveau problème technique consistant en la fourniture d'un procédé et d'un appareil plus simples, d'un réglage et d'une maintenance faciles, réduisant l'exposition du patient aux rayonnements, ce qui est particulièrement important dans le cas de rayonnements X et permettant d'occuper un empâtement minimum au stockage et au transport.

La présente invention a encore pour but de résoudre les problèmes techniques énoncés ci-dessus, avec un minimum de manipulations, notamment un minimum d'étapes, ou de prises de vue ou de déplacements, en limitant ainsi également la dose d'exposition au rayonnement émis par la source de rayonnement, ce qui est particulièrement important lorsque la source de rayonnement émet des rayons X.

La présente invention a encore pour but de résoudre les nouveaux problèmes techniques énoncés ci-dessus d'une manière telle que la solution retenue permette d'utiliser le procédé et l'appareil de l'invention dans le cadre d'un appareil de traitement de cible, de préférence par ondes de pression, encore de préférence cette cible étant choisie parmi le groupe consistant d'une lithiase, par exemple rénale ou biliaire ; ce tissus, par exemple de tumeurs bénignes ou malignes ; et d'os, par exemple une zone osseuse, telle qu'une fracture ou une zone d'ostéoporose.

La présente invention permet de résoudre pour la première fois ces problèmes techniques de manière simultanée, particulièrement simple, peu coûteuse et utilisable à l'échelle industrielle et médicale.

Ainsi, selon un premier aspect, la présente invention fournit un procédé pour déterminer la position exacte d'une cible (C) par rapport à un point de référence (0) déterminé par exemple par un dispositif de traitement de la cible (C), comprenant l'emploi d'un dispositif d'émission comorenant une source oe rayonnement émettant un rayonnement de formation d'image au moins de la cible (C) capable d'être reçu par un dispositif de réception de ce rayonnement, ladite source et ledit dispositif de réception étant disposés de part et d'autre de ladite cible (C), ladite source étant de position connue par rapport au point de référence (0), et en ce ou'on détermine la position de la cible (C) à partir d'au moins une image de ladite cible (C), caractérisé en ce qu'on prévoit le dispositif de réception sous forme d'une barrette comprenant une partie active linéaire formée d'une multiplicité d'éléments discrets (e₁ - eₙ) sensibles au rayonnement dont les positions dans l'espace sont connues, et en ce qu'on réalise au moins un déplacement de la source de rayonnements et le dispositif de réception simultanément, sur une distance connue permettant d'émettre un rayonnement couvrant au moins la zone de la cible selon un déplacement choisi parmi une rotation autour d'un axe non perpendiculaire à la ligne définie par ladite partie active linéaire, et une translation, en obtenant ainsi au moins une image de la cible (C), et en ce qu'on détermine la position de la cible (C) à partir de ladite image et de ladite distance de déplacement connue.

Selon un mode de réalisation avantageux, on réalise le déplacement précité en translation pour au moins deux orientations ou inclinaisons différentes de la source de rayonnement et du dispositif de réception associé.

Selon un autre mode de réalisation avantageux, on déplace simultanément la source de rayonnement et le dispositif de réception en rotation selon un axe de rotation sensiblement parallèle à la ligne définie par la partie active linéaire précitée.

Selon un autre mode de réalisation de l'invention, la source de rayonnement et le dispositif de réception sont montés sur un dispositif support commun unique, de préférence en forme de bras en C dont une extrémité est disposée au-dessus de la cible et dont l'autre extrémité est disposée en dessous de la cible. Avantageusement, le disoositif support commun est monté en rotation autour d'un axe de rotation avantageusement disposé sensiblement perpendiculairement à l'axe longitudinal de l'aopareil qui est habituellement défini par l'axe longitudinal d'une table dite de travail, de support d'un patient allongé sur ladite table selon le même axe longitudinal ; cet axe de rotation étant de préférence monté parallèle à la surface active sensible du dispositif de réception.

Selon une variante de réalisation particulière, on déplace en translation le dispositif support commun dans une direction sensiblement perpendiculaire à l'axe de rotation qui est lui-même monté parallèle à la surface active sensible du dispositif de réception.

Selon une autre variante de réalisation particulière, on déplace en translation le dispositif support commun sur une distance permettant d'émettre un rayonnement couvrant au moins la zone de la cible ; ce déplacement en translation étant réalisé pour au moins deux orientations ou inclinaisons différentes du dispositif support commun obtenues par une simple mise en rotation autour de l'axe de rotation.

Selon un mode de réalisation particulier, le dispositif support commun est monté en rotation sur un élément support faisant partie d'un chariot monté déplaçable en translation sur au moins un, ou de préférence deux rails de guidage en translation solidaires du châssis de l'appareil.

Selon un autre mode de réalisation, la source est une source de rayonnement X et le dispositif de réception précité comprend des éléments discrets sensibles au rayonnement X.

Selon encore un autre mode de réalisation de l'invention, la source précitée est une source de rayonnement X et le dispositif de rayonnement précité comprend au moins un écran fluorescent sensible aux rayons X et des éléments discrets sensibles au rayonnement fluorescent de cet écran, par exemple des éléments discrets sensibles aux photons lumineux.

Selon un autre mode de réalisation, les éléments discrets comprennent des composants à l'état solide et sensibles au rayonnement X, par exemple à éléments semi-conducteurs.

Selon un autre mode de réalisation, le procédé réalise le déplacement du dispositif de réception selon une vitesse de déplacement connue prédéterminée, ce qui permet de déterminer la position du dispositif de réception à un moment donné.

Selon un autre mode de réalisation, le procédé détermine quel est l'élément discret qui a reçu l'image de la cible (C), pour chacune des deux images résultant de deux positions angulaires différentes de la source de rayonnement, et à partir de la connaissance de la position exacte des deux éléments discrets ayant reçu les deux images de la cible C résultant des deux positions de la source de rayonnement, on détermine la position exacte dans l'espace de la cible.

Selon un autre mode de réalisation, le procédé prévoit un écran de contrôle sur lequel on projette au moins une image de la zone de la cible C obtenue lors du déplacement du dispositif de réception précité, ainsi que des moyens de pointage de la position de la cible C sur l'écran et des moyens de calcul permettant de calculer la position de la cible C avec les moyens de pointage, pour chaque image.

Selon un autre mode de réalisation, le procédé prévoit un dispositif de collimation permettant de limiter dans l'espace la zone de rayonnement, ce qui permet de limiter l'irradiation du patient ; de préférence déplacé simultanément avec la source de rayonnement et le dispositif de réception précités.

Selon encore un autre mode de réalisation, le procédé prévoit que le dispositif de réception comprend ladite partie active linéaire formée par une multiplicité d'éléments discrets alignés sensibles au rayonnement dont les positions dans l'espace sont connues, définissant un plan général de réception du rayonnement, on prévoit que la source d'émission du rayonnement émette ce rayonnement initialement dans une direction sensiblement perpendiculaire au plan de réception, ce rayonnement initialement perpendiculaire à ce plan étant renvoyé par un dispositif de renvoi pouvant éventuellement réémettre ce rayonnement, dans une direction parallèle au plan de réception, et on prévoit un dispositif de collimation comprenant une fente de collimation parallèle au plan de réception et limitant la zone de rayonnement sensiblement à la dimension sensiblement linéaire de la partie active du dispositif de réception. De préférence, la source d'émission du rayonnement est un tube à rayons X dont l'axe est avantageusement disposé sensiblement perpendiculairement à la direction du plan de réception. Grâce à l'invention, on minimise les variations d'intensité du rayonnement atteignant la zone sensible du dispositif de réception grâce au fait qu'il a été observé que le rayonnement est davantage uniforme lorsque la source a son axe disposé sensiblement perpendiculairement à la direction du plan de réception, après avoir été renvoyé dans ce plan par un dispositif de renvoi généralement dénommé cathode.

Grâce au fait que la source d'émission et le dispositif de réception sont liés mécaniquement en étant montés sur un dispositif support commun, on simplifie la conception de la structure de l'appareil et la mise en oeuvre du procédé de repérage, le réglage et la maintenance sont faciles. L'exposition du patient aux rayonnements est réduite, ce qui particulièrement intéressant dans le cas des rayons X. En outre, par le montage du dispositif support commun en rotation et également en translation, il est possible de dégager complètement le support commun pour l'allonger le long de l'appareil ce qui limite l'encombrement lors du stockage ou du transport, sans démontage. On peut aussi prérégler la fabrication du dispositif émetteur de rayonnement ainsi que le dispositif de réception.

Selon un deuxième aspect, la présente invention fournit un appareil pour déterminer la position exacte d'une cible (C) par rapport à un point de référence (0) déterminé par exemple par un dispositif de traitement de la cible C, comprenant un dispositif d'émission comprenant une source de rayonnement émettant un rayonnement de formation d'image au moins de la cible C capable d'être reçu par un dispositif de réception de ce rayonnement, ladite source et ledit dispositif de réception étant disposés de part et d'autre de ladite cible C, ladite source étant de position connue par rapport au point de référence (0) ; et par des moyens de détermination de la position de la cible C à partir d'au moins une image de ladite cible, caractérisé en ce que le dispositif de réception est réalisé sous forme d'une barrette comprenant une surface active linéaire formée d'une multiplicité d'éléments discrets (e₁ à eₙ) alignés sensibles au rayonnement dont les positions dans l'espace sont connues ; et des moyens de déplacement pour déplacer simultanément la source de rayonnement et le dispositif de réception, sur une distance de déplacement connue permettant d'émettre un rayonnement couvrant au moins la zone de la cible C pour obtenir au moins une image de ladite cible C; lesdits moyens de déplacement étant prévus pqur réaliscr un déplacement choisi parmi un déplacement en rotation autour d'un axe non-perpendiculaire à la ligne définie par ladite partie active linéaire et une translation ; lesdits moyens de détermination de la position de la cible C comprenant des moyens de calcul de la position de la cible C à partir de ladite image de la cible C et de la distance de déplacement connue.

Selon un mode de réalisation indépendamment brevetable, la source de rayonnement et le dispositif de réception sont montés sur un dispositif support commun, de préférence ayant une forme de bras en C dont les extrémités sont disposées de part et d'autre de la cible.

Selon un mode de réalisation particulier, le dispositif d'émission précité comprend des moyens de déplacement de la source de rayonnement entre deux positions angulaires différentes et des moyens de détermination de la position de la cible (C) à partir d'au moins deux images obtenues à partir de deux positions angulaires différentes de la source.

Selon encore un autre mode de réalisation, la source précitée est une source de rayonnement X et le dispositif de réception précité comprend des éléments discrets et sensibles au rayonnement X.

Selon encore une autre variante de réalisation de l'invention, la source est une source de rayonnement X et le dispositif de réception précité comprend au moins un écran fluorescent sensible aux rayons X et des éléments discrets sensibles au rayonnement fluorescent de cet écran, par exemple des éléments discrets et sensibles aux photons lumineux.

Selon encore un autre mode de réalisation, les éléments discrets comprennent des composants à l'état solide et sensibles au rayonnement X, par exemple à éléments semi-conducteurs.

Selon encore un autre mode de réalisation, des moyens de détermination de la position du dispositif de réception à un moment donné sont prévus pour déterminer quelle est la position exacte de l'élément discret qui a reçu l'image de la cible (C), et l'instant où cet élément a reçu l'image, pour chacune de deux images résultant de deux positions angulaires différentes de la source de rayonnement, ainsi que pour déterminer la position exacte dans l'espace de la cible (C) à partir de cette connaissance de la position exacte des deux éléments discrets ayant reçu les deux images de la cible C résultant des deux positions de la source de rayonnement.

Selon une variante de réalisation particulière, un écran de contrôle est prévu sur lequel au moins une image de la zone de la cible C obtenue lors du déplacement du dispositif de réception précité est projetée, ainsi que des moyens de pointage de la position de la cible C sur l'écran et des moyens de calcul permettant de calculer la position de la cible C avec les moyens de pointage, pour chaque image.

Selon encore un autre mode de réalisation, un dispositif de collimation est prévu, permettant de limiter dans l'espace la zone de rayonnement, en limitant ainsi l'irradiation du patient.

Selon encore un autre mode de réalisation, la source de rayonnement émet un rayonnement dans une direction initiale sensiblement perpendiculaire à un plan de réception défini par la partie active linéaire et l'axe de rotation précité, un dispositif de renvoi étant prévu pouvant réaliser une réémission de celui-ci dans le plan de réception, ainsi qu'un dispositif de collimation éventuel comprenant une fente disposée parallèlement au plan de réception et dans une direction perpendiculaire à la direction initiale du rayonnement.

Selon un troisième aspect, la présente invention couvre encore l'utilisation du procédé et/ou de l'appareil précité, dans le cadre d'un appareil de traitement d'une cible, de préférence par ondes de pression. Cet appareil de traitement est en particulier un appareil de traitement d'une cible choisie parmi le groupe consistant d'une lithiase, par exemple une lithiase rénale ou biliaire ; de tissus, par exemple des tumeurs bénignes ou malignes ; d'os, par exemple une fracture ou une zone d'ostéoporose. Un appareil de traitement préféré est caractérisé en ce qu'il comprend un réflecteur ellipsoidal tronqué rempli d'un liquide de couplage comprenant un foyer interne immergé dans ledit liquide et un foyer externe destiné à être mis en coïncidence avec la cible à traiter, ainsi qu'au moins deux électrodes disposées symétriquement de part et d'autre du foyer interne pour générer lesdites ondes de pression au foyer interne par décharge électrique dans ledit liquide de couplage.

On comprend que l'invention permet de résoudre les nouveaux problèmes techniques précédemment énoncés, d'aboutir aux avantages techniques déterminant également précédemment énoncés, ainsi que ceux qui apparaîtront clairement à l'homme de l'art à partir de la description suivante de l'invention qui va être faite en référence à un mode de réalisation actuellement préféré donné simplement à titre d'illustration et qui ne saurait donc en aucune façon limiter la portée de l'invention. Dans les dessins :
- la figure 1 est une vue schématique de principe d'un appareil de traitement d'une cible C à traiter, par exemple ici une lithiase rénale à l'intérieur du rein d'un patient ;
- la figure 2 représente une vue schématique selon la flèche II de la figure 3 ;
- la figure 3 représente une vue schématique selon la flèche III de la figure 2 montrant la réduction du volume du rayonnement grâce à l'emploi d'un dispositif de collimation ;
- la figure 4A représente schématiquement le déplacement entre une disposition initiale et une position finale, de manière simultanée, de la source de rayonnement, du dispositif de collimation et du dispositif de réception du rayonnement, selon une vitesse de déplacement connue ;
- la figure 4B représente schématiquement l'inclinaison simultanée de la source de rayonnement, du dispositif de collimation et du dispositif de réception du rayonnement de façon à former deux images de la cible, par pivotement autour d'un axe de rotation ;
- la figure 5 représente schématiquement les positions réceptives au cours du temps permettant d'observer la position pour laquelle le rayonnement atteint la cible C ;
- la figure 6 représente une vue latérale en élévation schématique d'un appareil de traitement d'une cible équipé d'un appareil pour déterminer la position exacte d'une cible, ici comprenant un bras en forme de C pivotant autour d'un axe horizontal et déplaçable en translation ;
- la figure 7 représente une vue en coupe avec arrachement partiel selon l'axe longitudinal de l'appareil de la figure 6, ou encore selon la ligne de coupe VII-VII de la figure 8 ;
- la figure 8 représente une vue en coupe avec arrachement partiel selon la ligne de coupe VIII-VIII de la figure 7 ; et
- la figure 9 représente schématiquement et de manière agrandie la direction initiale du rayonnement, par exemple un faisceau de rayons X, par rapport au dispositif de collimation et au plan de réception du dispositif de réception comprenant une surface active linéaire formée d'une pluralité d'éléments de réception de rayonnement alignés.

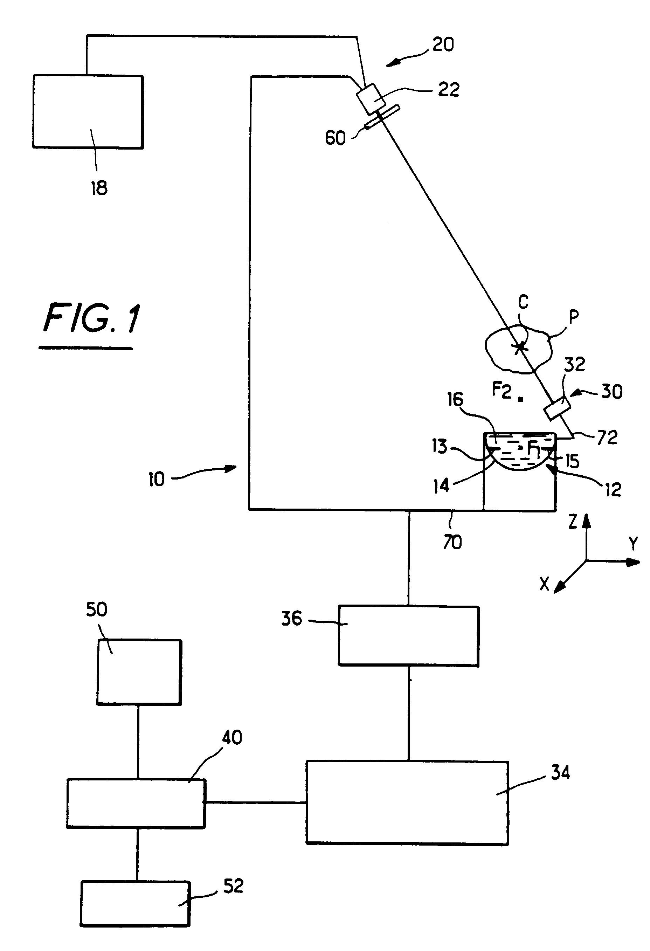

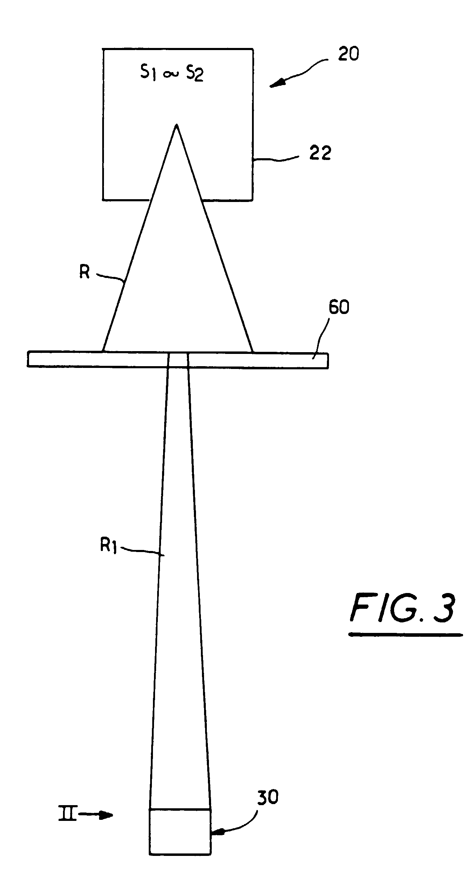

En référence aux figures 1 à 5, et plus particulièrement à la figure 1, un appareil de traitement selon la présente invention est représenté par le numéro de référence général 10. Cet appareil de traitement est de préférence un appareil de traitement émettant des ondes de pression, encore mieux des ondes de pression focalisées en un point focal F2, destiné à être mis en coïncidence avec la cible C à traiter. Ainsi, cet appareil comprend un dispositif 12 générateur d'ondes de pression. Selon un mode de réalisation préféré, tel que représenté, ce dispositif de génération d'ondes de pression 12 comprend un réflecteur ellipsoidal tronqué 14 rempli d'un liquide 16, qui est bien connu à l'homme de l'art et par exemple décrit dans le brevet US RIEBER 2 559 227 ou bien dans des documents antérieurs du déposant, tels que US-A-4 730 614 ; US-A-4 866 330 ; US-A-4 915 094 ; US-A-4 962 753 auxquels l'homme de l'art pourra se reporter. Ce réflecteur ellipsoidal tronqué 14 comprend un foyer interne F₁ et un foyer externe F₂ destiné à être mis en coïncidence avec la cible C à traiter. Ce réflecteur 14 est pourvu d'au moins deux électrodes 13, 15 disposées symétriquement relativement au foyer interne F₁ et générant par décharge électrique, à l'aide d'un générateur haute tension, des ondes de pression au foyer F₁, qui sont focalisées au foyer externe F₂ en étant transmises par le liquide de couplage 16, comme cela est bien connu à l'homme de l'art. Le dispositif 12 est habituellement monté déplaçable dans l'espace dans les trois directions X, Y, Z, grâce à des moyens de commande 34, par exemple avec des moteurs 36.

L'appareil selon l'invention 10 comprend un dispositif d'émission 20 comprenant une source de rayonnement 22 émettant un rayonnement de formation d'image au moins de la cible C capable d'être reçu par un dispositif de réception 30 de ce rayonnement. La source 22 et le dispositif de réception 30 sont disposés de part et d'autre de la cible C. D'autre part, la source 22 est de position connue par rapport au point de référence 0. Dans le cadre d'un exemple de réalisation préféré, le point de référence O est constitué par le foyer externe F₂ du réflecteur ellipsoidal tronqué 14.

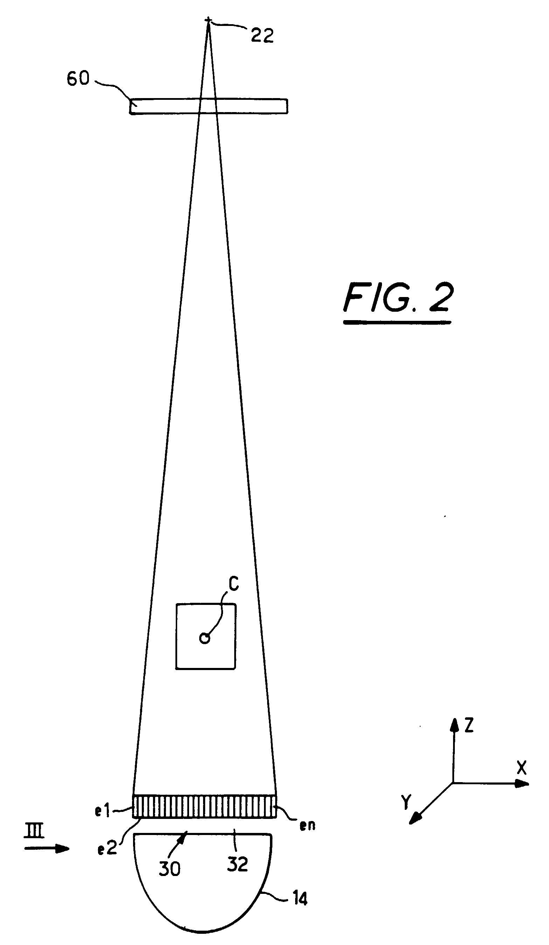

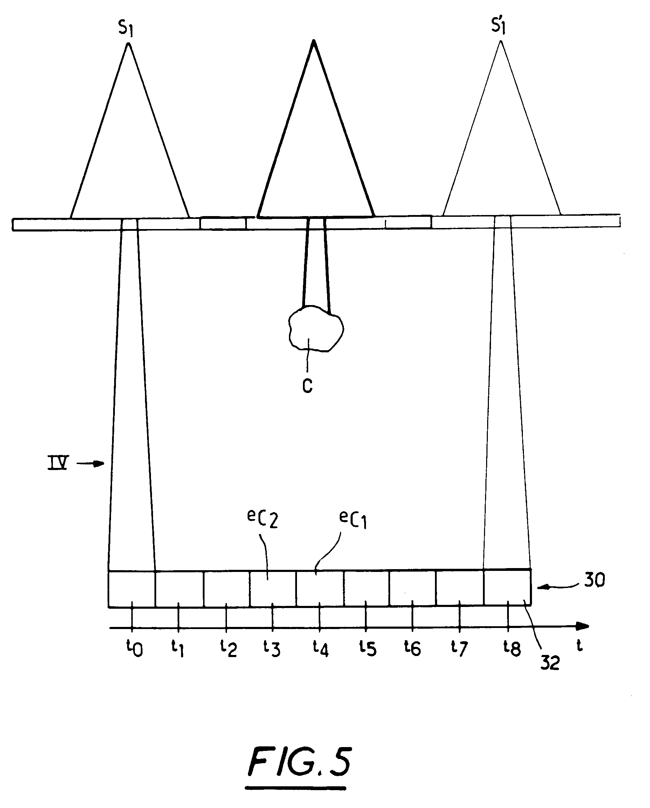

Selon la présente invention, l'appareil est caractérisé en ce que le dispositif de réception 30 comprend une barrette 32 comprenant une partie active linéaire formée d'une multiplicité d'éléments discrets e₁, e₂..., eₙ alignés, sensibles aux rayonnements dont on connaît les positions dans l'espace que l'on voit particulièrement bien aux figures 2 et 5.

D'autre part, cet appareil comprend aussi des moyens 40 de détermination de la position de la cible C à partir d'au moins une et de préférence deux images obtenues à partir de deux positions angulaires différentes S₁, S₂ de la source 22. L'appareil comprend donc également des moyens (non représentés) de déplacement de la source 22, qui sont bien connus de l'homme de l'art.

Selon une caractéristique particulière, le dispositif de réception 30 est réalisé sous forme d'une barrette 32, des moyens de déplacement 34 de ladite barrette 32 étant prévus pour déplacer ladite barrette 32 de manière à couvrir au moins l'ensemble de la zone de la cible C.

Selon une variante de réalisation, la source 22 précitée est une source de rayonnement X et le dispositif de réception 30 comprend des éléments discrets, référencés e₁, e₂..., eₙ à la figure 2, qui sont sensiblesau rayonnement X, de préférence disposés parallèles et alignés pour définir une surface active linéaire de réception.

Selon encore une autre variante de réalisation de l'invention, la source 22 est une source de rayonnement X et le dispositif de réception 30 comprend au moins un écran fluorescent sensible aux rayons X et des éléments discrets, référencés e₁, e₂..., eₙ, figure 2, sensibles au rayonnement fluorescent de cet écran, par exemple des éléments discrets sensibles aux photons lumineux.

Selon un autre mode de réalisation, les éléments discrets e₁ à eₙ comprennent des composants à l'état solide et sensibles au rayonnement X, par exemple à éléments semiconducteurs.

Selon encore un autre mode de réalisation de l'invention, des moyens de déplacement 34 du dispositif de réception 30 sont prévus, capables de réaliser un déplacement du dispositif de réception 30 selon une vitesse de déplacement connue prédéterminée, ainsi que des moyens de calcul 40, comprenant par exemple un calculateur, ordinateur ou micro-ordinateur, permettant de calculer la position du dispositif de réception 30 à un moment donné à partir du temps de déplacement et de la vitesse de déplacement connus, comme cela se conçoit bien à partir de la considération de la figure 5 qui montre le mouvement de déplacement de la barrette 32 en fonction du temps au cours du déplacement. Les positions de la barrette 32 sont représentées t₀, t₁, t₂ jusqu'à t₈ par exemple.

Selon encore un autre mode de réalisation, des moyens de détermination de la position du dispositif de réception 30 à un moment donné sont prévus pour déterminer quel est l'élément discret parmi les éléments e₁ à eₙ, qui a reçu l'image de la cible C, et à quel instant t, pour chacune des deux images résultant de deux positions angulaires différentes S₁, S₂, représentées à la figure 48 de la source 22 de rayonnement, ainsi que pour déterminer la position exacte dans l'espace de la cible C à partir de la connaissance de la position exacte des deux éléments discrets ayant reçu les deux images de la cible C résultant des deux positions S₁, S₂ de la source 22 de rayonnement. Ces moyens de détermination sont de préférence intégrés dans les moyens de calcul précités 40.

Selon une autre variante de réalisation particulière, un écran de contrôle 50 est prévu sur lequel au moins une image de la cible C obtenue lors du déplacement du dispositif de réception 30 est projetée, ainsi que des moyens 52 tels qu'un clavier ou une souris, bien connus de l'homme de l'art, permettant de réaliser le pointage de la position de la cible C sur l'écran 50 et des moyens de calcul 40 permettant de calculer la position de la cible C avec les moyens de pointage 52 pour chaque image.

Selon encore un autre mode de réalisation, un dispositif 60 de collimation est prévu, permettant de limiter dans l'espace la zone de rayonnement, en limitant ainsi l'irradiation du patient.

Selon un autre mode de réalisation, des moyens, symbolisés ici 70, 72 sont prévus pour déplacer simultanément la source de rayonnement 22, le dispositif de collimation 60 éventuel et le dispositif de réception 30, afin de limiter grandement le volume et le temps d'irradiation. Ces moyens de déplacement réalisent avantageusement soit un déplacement en translation, soit en rotation selon une direction non perpendiculaire à la ligne définie par la partie active linéaire formée par les éléments sensibles e₁ à eₙ.

On conçoit également qu'avec l'appareil qui vient d'être décrit une image de la zone contenant la cible peut être obtenue avec une source immobile pendant la formation de chaque image, dans la mesure où l'on prévoit des moyens de déplacement du dispositif de réception. Ainsi, dans ce cas, seul le déplacement du dispositif de réception apparaît essentiel.

C'est pourquoi selon une variante de réalisation dans le cas où la source est immobile pendant la formation de chaque image, des moyens 70, 72 seront prévus pour déplacer le dispositif de réception 30 et en synchronisme le dispositif de collimation 60, de manière que le rayonnement ne couvre sensiblement que ledit dispositif de réception, ce qui limite grandement le volume et le temps d'irradiation.

L'appareil dans son ensemble qui vient d'être décrit peut constituer un appareil de traitement d'une cible C, de préférence ici par ondes de pression. Cette cible est en particulier choisie parmi le groupe consistant d'une lithiase, par exemple une lithiase rénale ou biliaire ; de tissus, par exemple des tumeurs bénignes ou malignes ; d'os, par exemple une fracture ou une zone d'ostéoporose.

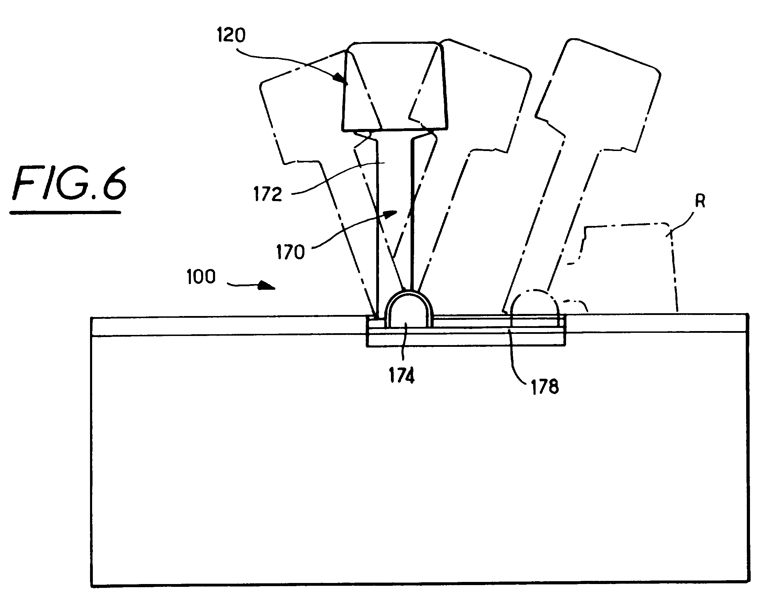

Un tel appareil fait l'objet plus précisément des figures 6 à 9 qui seront décrites plus loin, avec un autre mode de réalisation d'un appareil de détermination de la position d'une cible.

La procédure de détermination de la position de la cible C résulte de la description précédente faite en référence aux figures 1 à 5, et est la suivante :

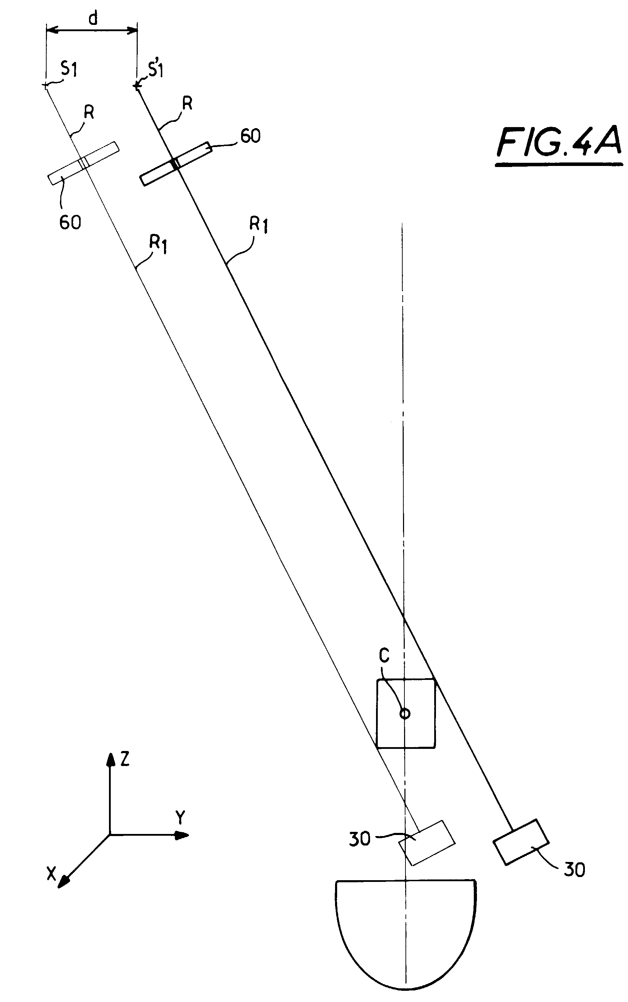

Tout d'abord, en partant de la position S₁ de la source 22 représentée en trait fantôme à la figure 4A, position pour laquelle par exemple la source S₁ est inclinée d'environ 20° par rapport à l'axe de symétrie Z-Z, représenté en trait mixte, du dispositif de traitement 12, coïncidant avec l'axe de déplacement selon la direction Z, on fait émettre un premier rayonnement par la source S₁ englobant la cible C et on fait déplacer le dispositif de réception 30 qui est par exemple disposé selon l'axe X, dans la direction de déplacement Y, c'est-à-dire perpendiculaire au plan X-Z, avantageusement selon une vitesse de déplacement en translation connue.

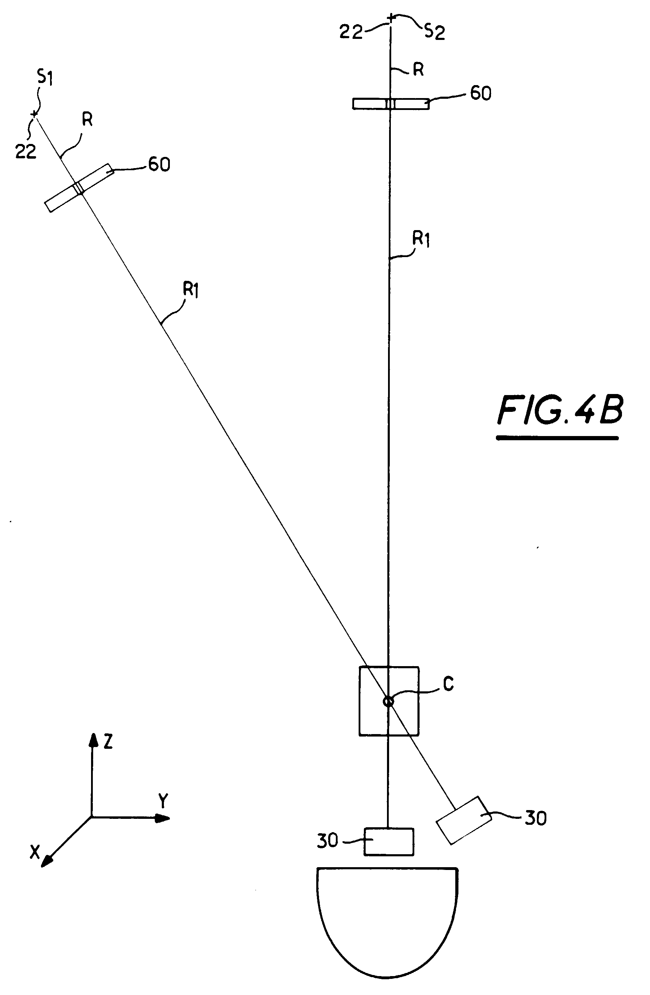

Selon le mode de réalisation préféré, tel que représenté aux figures 4A, 4B et 5 où la source S₁ se déplace simultanément au dispositif de réception 30, éventuellement lui-même solidaire du dispositif de traitement 12, et éventuellement avec déplacement également simultané du dispositif de collimation 60, le rayonnement (R) de la source, ici en position S₁, est limité (R₁) à la dimension du dispositif de réception 30, c'est-à-dire à la dimension de la barrette 32 pour couvrir l'ensemble des éléments discrets e₁ à eₙ. La distance du déplacement d entre la position en trait fantôme et la position en trait fort du dispositif de traitement, selon la direction Y, est clairement visible à la figure 4A. On observe que la source 22 est passée de la position S₁ à la position S'₁ en étant passée en regard de la cible C. Lors du passage en regard de la cible C, le rayonnement a été arrêté par la cible C comme représenté à la figure 5. Il est donc possible de détecter quel élément, par exemple ici appelé e_{C1}, n'a pas reçu le rayonnement émis par la source 22 en position S₁ à l'instant ici marqué t₄ qui référence l'instant où la source est passée en regard de la cible C pour la position S₁, ce qui est noté par les moyens de calcul 40.

Après ce premier passage, on peut modifier la position de la source 22 pour la disposer dans la position S₂, avec une inclinaison différente par rapport au dispositif de réception et de la cible C. Cette position S₂ est par exemple une inclinaison d'environ 20° de l'autre côté de la verticale Z-Z passant par la cible C.

On peut à ce moment là effectuer un déplacement de la distance d en sens inverse de manière qu'à un moment donné, un autre élément discret du dispositif de réception 30, par exemple un élément référencé e_{C2}, ne reçoive pas le rayonnement de la source 22 lorsqu'il est arrêté par la cible C en fournissant ainsi une deuxième image de la cible C pour une inclinaison différente de la source 22, dans la position S₂.

Les données de l'instant du déplacement, en fonction de la vitesse de déplacement, ainsi que de la position de l'élément discret e_{C2} n'ayant pas reçu le rayonnement de la cible C, sont transmises aux moyens de calcul 40.

Les moyens de calcul 40 permettent alors de calculer la position exacte de la cible C de manière simple.

Le praticien peut participer à cette procédure en pointant lui-même avec les moyens de pointage 52 l'image de la cible C sur les moyens formant écran 50, ce qui permet aussi aux moyens de calcul 40 d'effectuer rapidement le calcul exact de la position de la cible C dans l'espace relativement à une position initiale de référence du dispositif de traitement 12. Cette position initiale, ou point de référence 0, peut par exemple être la position initiale du foyer F₂ externe ou point de focalisation des ondes de pression du dispositif de traitement 12.

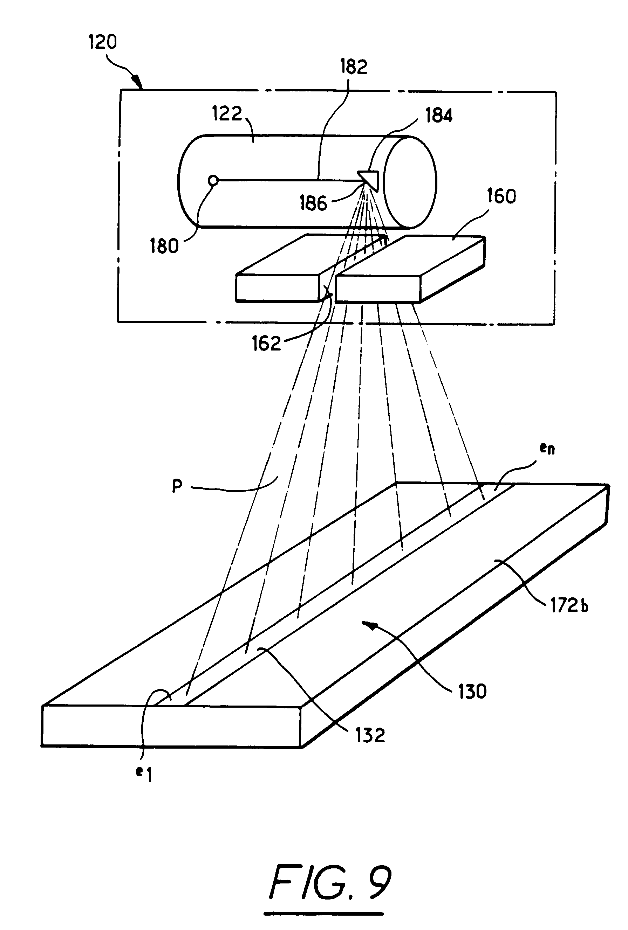

En référence à la figure 6, on a représenté un second mode de réalisation d'un appareil de traitement selon l'invention représenté par le numéro de référence général 100. Comme il est usuel, pour les organes remplissant les mêmes fonctions que ceux décrits dans le cadre du mode de réalisation des figures 1 à 5, on utilise le même numéro de référence augmenté de 100. Ainsi, le générateur d'ondes de pression est référencé 112, le dispositif d'émission est référencé 120 et le dispositif de réception est référencé 130. Ainsi, la source elle-même est référencée 122. Le dispositif de réception 130 a sa partie active aussi linéaire et subdivisée en une multiplicité d'éléments discrets alignés e₁ à eₙ identiques à ceux du mode de réalisation précédent, et dont le premier e₁ et le dernier eₙ ont été repérés à la figure 9. Autrement dit, le dispositif de réception 130 présente la forme d'une barrette 132 à partie active linéaire. Les moyens de déplacement de la barrette 132 sont ici référencés 170 en correspondant ainsi au moyen 70 de déplacement simultané prévu dans le mode de réalisation des figures 1 à 5. Les moyens de déplacement 170 permettent selon un mode de réalisation actuellement préféré, tel que représenté, un déplacement en translation, de préférence dans une direction sensiblement perpendiculaire à la partie active sensible du dispositif de réception 132. Selon un autre mode de réalisation, les moyens de déplacement permettent de réaliser un déplacement en rotation selon une direction non perpendiculaire à la ligne définie par ladite partie active linéaire 132.

Ces moyens de déplacement simultané 170 du dispositif d'émission 120 et du dispositif de réception 130 comprenant ici un bras en forme de C repéré 172 dont une première extrémité 172a supporte le dispositif d'émission 120 comprenant la source de rayonnement 122 et le dispositif de collimation 160 et une deuxième extrémité 172b supporte le dispositif de réception du rayonnement 130 intégrant ici la barrette 132 des éléments sensibles e₁ à eₙ. Ces moyens de déplacement 170 constituent ainsi un support commun de la source de rayonnement 122, du dispositif de collimation 160 et du dispositif de réception 130.

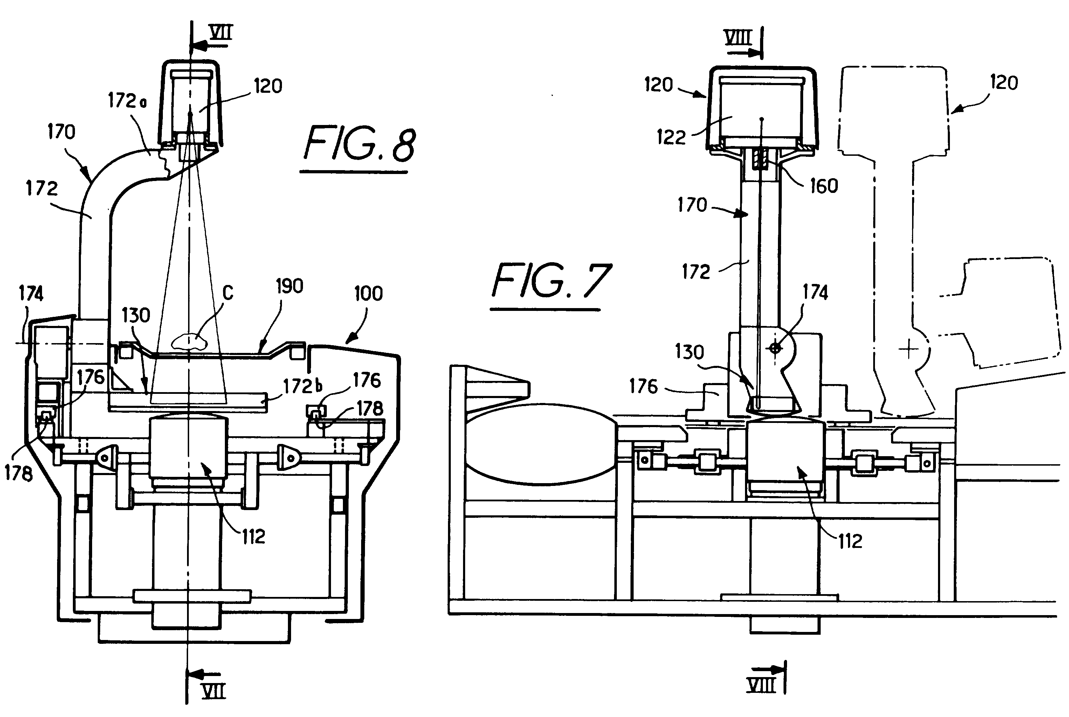

Selon un mode de réalisation avantageux, ces moyens de déplacement 170 formant support commun sont montés en rotation autour d'un axe de rotation 174 disposé sensiblement perpendiculairement à l'axe longitudinal de l'appareil 100 comme cela est clairement visible aux figures 6 à 8. Cet axe longitudinal correspond habituellement aussi à l'axe longitudinal d'une table 190 sur laquelle s'allonge le patient à traiter. Cette table 190 est de préférence amovible, notamment pour permettre un basculement complet des moyens de déplacement 170 dans la position de repos R représentée à la figure 7 permettant d'occuper un volume minimum lors du stockage et du transport.

Selon un autre mode de réalisation particulièrement avantageux, les moyens de déplacement 170 formant dispositif support commun sont montés sur un dispositif de translation 176 monté en coulissement par translation sur des rails de guidage 178, montés parallèlement à l'axe longitudinal de l'appareil. On comprend que les moyens de déplacement 170 formant dispositif support commun, ici en forme de bras 172 en C sont disposés latéralement par rapport à la table de travail 190 destinée à recevoir le patient et de façon que les extrémités 172a, 172b supportant respectivement le dispositif d'émission 120 et le dispositif de réception 130, soient disposées de part et d'autre de la table de travail 190, donc de part et d'autre du patient et de la zone cible C.

La structure des moyens de déplacement 170 fait partie intégrante de l'invention et est indépendamment brevetable.

D'autre part, l'invention prévoit aussi de manière indépendamment brevetable la conception du dispositif d'émission 120 en prévoyant que la source d'émission 122 comprenne un élément générateur 180 émettant un rayonnement 182 dont la direction initiale est sensiblement perpendiculaire au plan P de réception défini par la surface active sensiblement linéaire de la barrette 132 formée par les éléments e₁ à eₙ de réception de rayonnement. Le rayonnement initialement perpendiculaire 182 est renvoyé dans la direction du plan P par un dispositif de renvoi ou de réémission tel qu'une cathode 184 comportant une face inclinée 186 assurant ce renvoi ou cette réémission du rayonnement 182. La source de rayonnement préférée est selon l'invention une source de rayonnement X comprenant un tube à rayon X dont l'axe est avantageusement disposé sensiblement perpendiculairement à la direction du plan de réception P, et dont au moins une partie est réémise par le dispositif de renvoi formé ici par une cathode 184. Le dispositif de collimation 160 présente une fente de collimation 162 disposée parallèlement au plan P, et donc aussi perpendiculairement à la direction initiale du rayonnement 182, et dont la dimension est prévue pour fournir un rayonnement dont la dimension est sensiblement voisine, ou légèrement supérieure, à la dimension de la partie active sensiblement linéaire formant barrette 132 du dispositif de réception 130.

De cette manière, on minimise les variations d'intensité du rayonnement atteignant la partie active de la barrette 132, de sorte que leur effet devient négligeable sur la qualité de l'image finalement obtenue, en raison de la dimension sensiblement linéaire de la barrette. Cette barrette 132 peut, par exemple, présenter environ 0,6 mm de large sur 230,4 mm de long. De ce fait, le dispositif de collimation 160 va produire un faisceau sensiblement plan très fin traversant le patient en direction de la barrette ce qui minimisera la quantité de rayonnements reçus par le patient, ce qui est particulièrement important dans le cas préféré de l'emploi de rayons X. D'autre part, en prévoyant la dimension de la fente 162 du dispositif de collimation 160 pour que la largeur du faisceau plan P au niveau de la barrette 132 soit sensiblement égale ou légèrement supérieure à celle de la surface acquise de la barrette 132, la totalité du rayonnement qui traverse le patient est utile pour la formation d'image.

D'autre part, grâce à la prévision des moyens de déplacement 172 formant dispositif support commun de la source d'émission 120 et du dispositif de réception 130 et éventuellement du dispositif de collimation 160, on réalise une liaison mécanique et indéformable de ces dispositifs, ce qui garantit, par un réglage unique initial, que le rayonnement arrive toujours sur la partie active de la barrette 132 du dispositif de réception 130 quel que soit le déplacement ou l'orientation de l'ensemble. En outre, l'intensité du rayonnement reçu sera constante, il n'y a donc pas de risque de variation d'intensité du rayonnement d'une ligne à l'autre lors du balayage de la zone à imager.

Pour former une image, les moyens de déplacement 170 sont translatés grâce au moyen de translation 176 le long du patient en regard de la zone à imager. De préférence, et comme dans le cas précédent relatif aux figures 1 à 5, on réalise au moins deux images avec deux inclinaisons différentes des moyens de déplacement 170, procurant deux orientations angulaires différentes de la source de rayonnement 120. La première orientation peut, par exemple, être verticale comme représenté aux figures 6 à 8 et la deuxième position peut être inclinée à gauche ou à droite à partir de la position verticale comme représenté à la figure 6, avec une position translatée représentée en trait mixte, de manière à former deux images.

Selon une variante de réaliation particulièrement avantageuse, l'axe de rotation 174 est disposé à mi-hauteur de la zone à imager.

On notera qu'avec l'invention on peut prérégler la position initiale du plan P du faisceau émis par la source 122 ainsi que celle de la partie active de la barrette 132. De cette manière, les moyens de déplacement formant dispositif support commun 170 ici en forme de bras C 172 peuvent être fabriqués avec précision, le montage de n'importe quelle barrette 132 préréglée et de n'importe quelle source de rayonnement 122 préréglée permettent d'assurer un bon alignement du faisceau sur la zone active linéaire de la barrette 132. De ce fait, on peut prévoir à l'intérieur des extrémités 172a, 172b du bras, des mécanismes de réglage précis de position respectivement de la source, par exemple par réglage de position du dispositif de renvoi 184 ou de la barrette 132 intégrée à l'extrémité 172b. On peut également prérégler la position du dispositif de collimation 160, celui-ci étant aussi intégré dans l'extrémité 172a du bras 172.

Une fois ces réglages effectués, les positions sont rigidement bloquées. Dans le cas de la barrette 132, celle-ci peut être montée sur un cadre permettant un déplacement en X et Y et un blocage dans la position recherchée sur l'extrémité 172b du bras 172.

Il est ainsi clair que l'invention permet, d'une manière simple, d'aboutir aux avantages techniques déterminants précédemment énoncés.

Les modes de réalisation des figures 1 à 9 sont partie intégrante de la présente invention et sont donc partie intégrante de la présente description. L'invention couvre également toute caractéristique qui apparaîtra nouvelle vis-à-vis d'un état de la technique quelconque résultant de la description précédente intégrant les figures 1 à 9.

## Revendications

1. Procédé pour déterminer la position exacte d'une cible (C) par rapport à un point de référence (O) déterminé par exemple par un dispositif de traitement (12 ; 112) de la cible (C), comprenant l'emploi d'un dispositif d'émission (20 ; 120) comprenant une source (22 ; 122) de rayonnement émettant un rayonnement de formation d'image au moins de la cible C capable d'être reçu par un dispositif de réception (30 ; 130) de ce rayonnement, ladite source (22 ; 122) et ledit dispositif de réception (30 ; 130) étant disposés de part et d'autre de ladite cible (C), ladite source (22 ; 122) étant de position connue par rapport au point de référence (O), et en ce qu'on détermine la position de la cible (C) à partir d'au moins une image de ladite cible (C), caractérisé en ce que :
- on prévoit le dispositif de réception (30 ; 130) sous forme d'une barrette (32 ; 132) comprenant une partie active linéaire formée d'une multiplicité d'éléments discrets (e₁ à eₙ) sensibles au rayonnement dont les positions dans l'espace sont connues,
- on réalise au moins un déplacement de la source de rayonnements (22 ; 122) et du dispositif de réception (30 ; 130) simultanément, sur une distance connue permettant d'émettre un rayonnement couvrant au moins la zone de la cible (C) selon un déplacement choisi parmi une rotation autour d'un axe non perpendiculaire à la ligne définie par ladite partie active linéaire, et une translation, en obtenant ainsi au moins une image de la cible (C), et
- on détermine la position de la cible (C) à partir de ladite image et de ladite distance de déplacement connue.

2. Procédé selon la revendication 1, caractérisé en ce qu'on réalise le déplacement précité en translation pour au moins deux orientations ou inclinaisons différentes de la source de rayonnement et du dispositif de réception associé.

3. Procédé selon la revendication 1 ou 2, caractérisé en ce qu'on déplace simultanément la source de rayonnement (22 ; 122) et le dispositif de réception (30 ; 130) en rotation selon un axe de rotation sensiblement parallèle à la ligne définie par la partie active linéaire précitée.

4. Procédé selon l'une des revendications 1 à 3, caractérisé en ce qu'on monte la source de rayonnement (120) et le dispositif de réception (130) sur un dispositif support commun unique (170), de préférence en forme de bras en C (172) dont une extrémité (172a) est disposée au-dessus de la cible et dont l'autre extrémité (172b) est disposée en dessous de la cible.

5. Procédé selon la revendication 4, caractérisé en ce qu'on monte le dispositif support commun (170) en rotation autour d'un axe de rotation (174) avantageusement disposé sensiblement perpendiculairement à l'axe longitudinal de l'appareil qui est habituellement défini par l'axe longitudinal d'une table (190) dite de travail, de support d'un patient allongé sur ladite table selon le même axe longitudinal ; cet axe de rotation (174) étant de préférence monté parallèle à la surface active sensible (132) du dispositif de réception (130).

6. Procédé selon la revendication 5, caractérisé en ce qu'on déplace en translation le dispositif support commun (170) dans une direction sensiblement perpendiculaire à l'axe de rotation (174) qui est lui-même monté parallèle à la surface active sensible (132) du dispositif de réception (130).

7. Procédé selon la revendication 5 ou 6, caractérisé en ce qu'on déplace en translation le dispositif support commun (170) sur une distance permettant d'émettre un rayonnement couvrant au moins la zone de la cible (C) ; ce déplacement en translation étant réalisé pour au moins deux orientations ou inclinaisons différentes du dispositif support commun (170) obtenues par une simple mise en rotation autour de l'axe de rotation (174).

8. Procédé selon l'une des revendications 1 à 7, caractérisé en ce que la source de rayonnement de formation d'image est une source de rayonnement X et le dispositif de réception (30, 130) précité comprend des éléments discrets (e₁ à eₙ) sensibles au rayonnement X.

9. Procédé selon l'une des revendications 1 à 7, caractérisé en ce que la source de rayonnement est une source de rayonnement X et le dispositif de réception (30, 130) précité comprend au moins un écran fluorescent sensible aux rayons X et des éléments discrets (e₁ à eₙ) sensibles au rayonnement fluorescent de cet écran, par exemple des éléments discrets sensibles aux photons lumineux.

10. Procédé selon la revendication 8 ou 9, caractérisé en ce que les éléments discrets (e₁ à eₙ) comprennent des composants à l'état solide et sensibles au rayonnement X, par exemple à éléments semi-conducteurs.

11. Procédé selon l'une des revendications 1 à 10, caractérisé en ce qu'on réalise le déplacement du dispositif de réception selon une vitesse de déplacement connue prédéterminée ; en ce qu'on détermine quel est l'élément discret qui a reçu l'image de la cible (C), pour chacune de deux images résultant de deux positions angulaires différentes (S₁ ; S₂) de la source (22) de rayonnement, et qu'à partir de la connaissance de la position exacte des deux éléments discrets (e_{C1} ; e_{C2}, respectivement), ayant reçu les deux images de la cible (C) résultant des deux positions (S₁, S₂) de la source de rayonnement, on détermine la position exacte dans l'espace de la cible (C).

12. Procédé selon l'une des revendications précédentes, caractérisé en ce qu'on prévoit un écran de contrôle (50) sur lequel on projette au moins une image de la zone de la cible C obtenue lors du déplacement du dispositif de réception (30) précité, ainsi que des moyens de pointage (52) de la position de la cible C sur l'écran (50) et des moyens de calcul (40) permettant de calculer la position de la cible C, notamment avec les moyens de pointage (52), pour chaque image.

13. Procédé selon l'une des revendications précédentes, caractérisé en ce qu'on prévoit un dispositif de collimation (60 ; 160) permettant de limiter dans l'espace la zone de rayonnement (R₁), ce qui permet de limiter l'irradiation du patient ; de préférence déplacé simultanément avec la source de rayonnement et le dispositif de réception précités.

14. Appareil pour déterminer la position exacte d'une cible (C) par rapport à un point de référence (0) déterminé par exemple par un dispositif de traitement (12, 112) de la cible (C), comprenant un dispositif d'émission (20, 120) comprenant une source (22, 122) de rayonnement émettant un rayonnement de formation d'image au moins de la cible (C) capable d'être reçu par un dispositif de réception (30, 130) de ce rayonnement, ladite source (22, 122) et ledit dispositif de réception (30 ; 130) étant disposés de part et d'autre de ladite cible (C), ladite source étant de position connue par rapport au point de référence (0) ; et par des moyens (40) de détermination de la position de la cible (C) à partir d'au moins une image de ladite cible, caractérisé en ce que le dispositif de réception (30, 130) est réalisé sous forme d'une barrette (32, 132) comprenant une surface active linéaire formée d'une multiplicité d'éléments discrets (e₁ à eₙ) alignés sensibles au rayonnement dont les positions dans l'espace sont connues ; et des moyens de déplacement (34, 36 ; 70, 72 ; 170) pour déplacer simultanément la source de rayonnement (22, 122) et le dispositif de réception (30 ; 130), sur une distance de déplacement connue permettant d'émettre un rayonnement couvrant au moins la zone de la cible (C) pour obtenir au moins une image de ladite cible (C); lesdits moyens de déplacement étant prévus pour réaliser un déplacement choisi parmi un déplacement en rotation autour d'un axe non-perpendiculaire à la ligne définie par ladite partie active linéaire et une translation ; lesdits moyens (40) de détermination de la position de la cible (C) comprenant des moyens de calcul de la position de la cible (C) à partir de ladite image de la cible (C) et de la distance de déplacement connue.

15. Appareil selon la revendication 14, caractérisé en ce que les moyens de déplacement (34, 36 ; 70, 72 ; 170) sont prévus pour déplacer simultanément la source de rayonnement (22, 122) et le dispositif de réception (30 ; 130) selon une vitesse de déplacement connue prédéterminée, lesdits moyens de calcul (40) calculant la position de ia cible (C) à un moment donné à partir du temps de déplacement et la vitesse de déplacement connus.

16. Appareil selon la revendication 15, caractérisé en ce que la source de rayonnement (120) et le dispositif de réception (130) sont montés sur un dispositif support commun (170), de préférence ayant une forme de bras en C (172) dont les extrémités sont disposées de part et d'autre de la cible (C).

17. Appareil selon la revendication 16, caractérisé en ce que le dispositif support commun (170) est monté en rotation autour d'un axe de rotation (174) disposé sensiblement perpendiculairement à l'axe longitudinal de l'appareil (100) en général défini par l'axe longitudinal d'une table (190) dite de travail, de support d'un patient lui-même disposé selon le même axe longitudinal.

18. Appareil selon la revendication 16 ou 17, caractérisé en ce que les moyens de déplacement (34, 36 ; 70, 72 ; 170) sont prévus pour réaliser un déplacement du dispositif support commun (170) en translation relativement au châssis de l'appareil (100) dans une direction sensiblement perpendiculaire à la surface active sensible (32, 132) du dispositif de réception (30, 130) sur ladite distance couvrant au moins la zone de la cible (C) ; de préférence comportant un dispositif de translation comprenant un chariot (176) monté en translation sur au moins un rail ou de préférence deux rails (178) de guidage de translation solidaires du châssis de l'appareil.

19. Appareil selon l'une des revendications 15 à 18, caractérisé en ce que les moyens (40) de détermination de la position du dispositif de réception (30; 130) à un moment donné sont prévus pour déterminer quel est l'élément discret (eC₁,eC₂) qui a reçu l'image de la cible C pour chacune de deux images résultant de deux positions angulaires différentes (S₁, S₂) de la source de rayonnement (22), ainsi que de déterminer la position exacte dans l'espace de la cible (C) à partir de la connaissance de cette position exacte des deux éléments discrets (e_{C1}, e_{C2}), ayant reçu les deux images de la cible C résultant des deux positions de la source de rayonnement.

20. Appareil selon l'une des revendications 15 à 19, caractérisé en ce qu'un écran de contrôle (50) est prévu sur lequel au moins une image de la zone de ia cible (C) obtenue lors du déplacement du dispositif de réception (30) est projetée, ainsi que des moyens de pointage (52) de la position de la cible C sur l'écran et des moyens de calcul (40) permettant de calculer la position de la cible C avec les moyens de pointage (52), pour chaque image.

21. Dispositif selon l'une des revendications 15 à 20, caractérisé en ce qu'un dispositif de collimation (60 ; 160) est prévu, permettant de limiter dans l'espace la zone de rayonnement (R₁), en limitant ainsi l'irradiation du patient.

22. Appareil selon l'une des revendications 17 à 21, caractérisé en ce que la source de rayonnement (180) émet un rayonnement dans une direction initiale (182) sensiblement perpendiculaire à un plan de réception (P) défini par la partie active linéaire (32 ; 132) et l'axe de rotation (174) précités, un dispositif de renvoi (184) pouvant réaliser une réémission de celui-ci dans le plan (P) de réception, ainsi qu'un dispositif de collimation (160) comprenant une fente (162) disposée parallèlement au plan de réception (P) et dans une direction perpendiculaires à la direction initiale du rayonnement.

23. Utilisation du procédé selon l'une des revendications 1 à 13 et/ou de l'appareil selon l'une des revendications 14 à 22, dans le cadre d'un appareil (10 ; 100) de traitement d'une cible (C) de préférence par ondes de pression.

24. Utilisation selon la revendication 23, caractérisée en ce que l'appareil de traitement est un appareil de traitement d'une cible (C) choisie dans le groupe consistant d'une lithiase, par exemple une lithiase rénale ou biliaire ; de tissus, par exemple des tumeurs bénignes ou malignes ; d'os, par exemple une fracture ou une zone d'ostéoporose.

25. Utilisation selon la revendication 23 ou 24, caractérisée en ce que l'appareil de traitement comprend un dispositif de traitement (12; 112) comprenant un réflecteur ellipsoïdal tronqué (14) rempli d'un liquide de couplage (16) comprenant un foyer interne (F₁) immergé dans ledit liquide, et un foyer externe (F₂) destiné à être mis en coïncidence avec la cible (C) à traiter, ainsi qu'au moins deux électrodes (13, 15) disposées symétriquement de part et d'autre du foyer interne (F₁) pour générer lesdites ondes de pression au foyer interne (F₁) par décharge électrique dans le liquide de couplage (16).

## Patentansprüche

1. Verfahren zur genauen Lagebestimmung eines Ziels (C) in Bezug auf einen Referenzpunkt (O), der zum Beispiel durch ein Gerät (12;112), mit dem das Ziel (C) behandelt wird, gegeben ist, wobei eine Sendeeinrichtung (20; 120) mit einer Strahlenquelle (22; 122) eingesetzt wird, von der Strahlung ausgeht, die ein Bild, zumindest vom Ziel C, erzeugt, das von einer Empfangseinrichtung (30; 130) empfangen werden kann, wobei die Strahlenquelle (22; 122) und die Empfangseinrichtung (30; 130) sich diesseits und jenseits des Ziels (C) befinden, die Position der Quelle (22; 122) in Bezug auf den Referenzpunkt (O) bekannt ist und die Lage des Ziels (C) ausgehend von mindestens einem Bild des besagten Ziels (C) bestimmt wird, dadurch gekennzeichnet, daß:
- die Empfangseinrichtung (30; 130) in der Form einer Leiste (32; 132) vorgesehen wird, die eine lineare Anordnung einer Vielzahl aktiver Einzelelemente (e₁ bis eₙ) aufweist, die für Strahlen, deren Positionen im Raum bekannt sind, empfindlich sind,
- mindestens eine gleichzeitige Positionsveränderung der Strahlenquelle (22; 122) und der Empfangseinrichtung (30;130) um einen gegebenen Abstand durchgeführt wird, der es ermöglicht, ein Strahlenbündel abzugeben, das mindestens den Bereich des Ziels (C) abdeckt, gemäß einer Verschiebung, die aus einer Rotation um eine Achse, die nicht senkrecht zu der durch die aktive lineare Anordnung definierten Line liegt, und einer Translation gewählt wird, wodurch mindestens ein Bild des Ziels (C) erhalten wird; und
- die Lage des Ziels (C) ausgehend von dem Bild und dem gegebenen Abstand bestimmt wird.

2. Verfahren nach Anspruch 1, dadurch gekennzeichnet, daß die genannte Positionsveränderung durch Translation der Strahlenquelle und der Empfangseinrichtung in mindestens zwei verschiedenen Richtungen oder Neigungen vorgenommen wird.

3. Verfahren nach Anspruch 1 oder 2, dadurch gekennzeichnet, daß man gleichzeitig die Strahlenquelle (22; 122) und die Empfangseinrichtung (30;130) durch Rotation um eine Drehachse verschiebt, die annähemd parallell zu der Linie verläuft, welche durch die vorgenannte lineare aktive Anordnung definiert ist.

4. Verfahren nach einem der Ansprüche 1 bis 3, dadurch gekennzeichnet, daß die Strahlenquelle (120) und die Empfangseinrichtung (130) auf einer einzigen gemeinsamen Halterung (170), vorzugsweise in Form eines C-Armes (172), montiert werden, von dem ein Ende (172a) oberhalb des Ziels und dessen anderes Ende (172b) unterhalb des Ziels angeordnet ist.

5. Verfahren nach Anspruch 4, dadurch gekennzeichnet, daß die gemeinsame Halterung (170) um eine Drehachse (174) drehbar montiert wird, die vorzugsweise im Wesentlichen senkrecht zur Längsachse des Geräts angeordnet ist, welche üblicherweise durch die Längsachse eines Arbeitstisches (190) zur Abstützung eines nach derselben Längsachse ausgestreckten Patienten definiert wird; wobei diese Drehachse (174) vorzugsweise parallell zur aktiven strahlenempfindlichen Oberfläche (132) der Empfangseinrichtung (130) angeordnet ist.

6. Verfahren nach Anspruch 5, dadurch gekennzeichnet, daß die gemeinsame Halterung (170) in einer zur Drehachse (174) im Wesentlichen senkrechten Richtung translatorisch verschoben wird, wobei die Drehachse ihrerseits parallell zur aktiven strahlenempfindlichen Oberfläche (132) der Empfangseinrichtung angeordnet ist.

7. Verfahren nach Anspruch 5 oder 6, dadurch gekennzeichnet, daß die Position der gemeinsamen Halterung (170) translatorisch um einen Abstand verschoben wird, der es ermöglicht, daß Strahlung abgegeben wird, die mindestens den Bereich des Ziels (C) abdeckt; wobei diese translatorische Verschiebung mindestens in zwei verschiedene Richtungen oder Neigungen der gemeinsamen Halterung (170) durchgeführt wird, die man durch einfache Rotation um die Drehachse (174) erhält.

8. Verfahren nach einem der Ansprüche 1 bis 7, dadurch gekennzeichnet, daß die Strahlenquelle zur Bilderzeugung eine Röntgenstrahlenquelle ist und die genannte Empfangseinrichtung (30, 130) röntgenstrahlenempfindliche Einzelelemente (e₁ bis eₙ) enthält.

9. Verfahren nach einem der Ansprüche 1 bis 7, dadurch gekennzeichnet, daß die Strahlenquelle eine Röntgenstrahlenquelle ist und die genannte Empfangseinrichtung (30, 130) mindestens einen röntgenstrahlenempfindlichen Fluoreszenzschirm und für die Fluoreszenzstrahlen dieses Schirms empfindliche Einzelelemente (e₁ bis eₙ), z. B. lichtquantenempfindliche Einzelelemente, enthält.

10. Verfahren nach Anspruch 8 oder 9, dadurch gekennzeichnet, daß die Einzelelemente (e₁ bis eₙ) röntgenstrahlenempfindliche Festkörperkomponenten, z. B. Halbleiterelemente, aufweisen.

11. Verfahren nach einem der Ansprüche 1 bis 10, dadurch gekennzeichnet, daß die Position der Empfangseinrichtung mit einer bekannten, vorbestimmten Verschiebungsgeschwindigkeit verändert wird; daß für beide Bilder, die aus zwei verschiedenen Winkelpositionen (S₁, S₂) der Strahlenquelle (22) hervorgehen, bestimmt wird, welches Einzelelement das Bild des Ziels (C) empfangen hat, und daß aufgrund der Kenntnis der genauen Position der beiden Einzelelemente (e_{c1} bzw. e_{c2}), die ausgehend von den zwei Positionen (S₁, S₂) der Strahlenquelle die beiden Bilder des Ziels (C) empfangen haben, die genaue Lage des Ziels (C) im Raum bestimmt wird.

12. Verfahren nach einem der vorhergehenden Ansprüche, dadurch gekennzeichnet, daß ein Kontrollschirm (50), auf den mindestens ein Bild des Bereichs des Ziels (C) projiziert wird, das man bei der Positionsveränderung der Empfangseinrichtung (30) erhält, sowie Mittel (52) zum Anzeigen der Position des Ziels C auf dem Schirm (50) und Rechenmittel (40) vorgesehen werden, mit denen die Position des Ziels C, insbesondere mit den Anzeigemitteln (52), für jedes Bild berechnet werden kann.

13. Verfahren nach einem der vorhergehenden Ansprüche, dadurch gekennzeichnet, daß eine Kollimatoreinrichtung (60; 160) vorgesehen wird, um die Zone der Strahlen (R₁) im Raum zu beschränken, wodurch die Strahlenbelastung des Patienten reduziert werden kann, und deren Position vorzugsweise gleichzeitig mit der genannten Strahlenquelle und Empfangseinrichtung verändert wird.

14. Vorrichtung zum Bestimmen der genauen Position eines Ziels (C) in Bezug auf einen Referenzpunkt (0), welcher zum Beispiel durch ein Gerät (12, 112), mit dem das Ziel (C) behandelt wird, gegeben ist, mit einer Sendeeinrichtung (20, 120) mit einer Strahlenquelle (22, 122), die eine Strahlung abgibt, um ein Bild zumindest des Ziels (C) zu erzeugen, die von einer Strahlenempfangseinrichtung (30, 130) aufgenommen werden kann, wobei die Quelle (22, 122) und die Empfangseinrichtung (30, 130) diesseits und jenseits des Ziels (C) angeordnet sind und die Position der Quelle in Bezug auf den Referenzpunkt (0) bekannt und Mitteln (40) zur Bestimmung der Position des Ziels (C) ausgehend von wenigstens einm Bild dieses Ziels; die Empfangseinrichtung (30, 130) in Form einer Leiste (32, 132) vorgesehen ist, welche eine lineare aktive Oberfläche aufweist, die durch eine Vielzahl ausgerichteter strahlungsempfindlicher Einzelelemente (e₁ bis eₙ) gebildet ist, deren Positionen im Raum bekannt sind; und durch Mittel (34, 36, 70, 72; 170) zur gleichzeitigen Positionsveränderung der Strahlenquelle (22, 122) und der Empfangseinrichtung (30, 130) um einen bekannten Verschiebungsabstand, der es ermöglicht, ein Strahlenbündel abzugeben, das zumindest die Zone des Ziels (C) abdeckt, um zumindest ein Bild des Ziels (C) zu erhalten, wobei die Mittel zur Positionsveränderung so vorgesehen sind, daß sie wahlweise eine Rotation um eine Achse, die nicht senkrecht zu der durch die genannte lineare aktive Anordnung definierten Linie liegt, und eine Translation durchführen können; wobei die Mittel zur Bestimmung der Position des Ziels (C) Mittel zur Berechnung der Position des Ziels (C), ausgehend von dem erwähnten Bild des Ziels (C) und dem bekannten Abstand der Positionsveränderung, enthalten.

15. Vorrichtung nach Anspruch 14, dadurch gekennzeichnet, daß die Mittel zur Positionsveränderung (34, 36, 70, 72; 170) so vorgesehen sind, daß sie gleichzeitig die Position der Strahlenquelle (22, 122) und der Empfangseinrichtung (30; 130) mit einer bekannten, vorbestimmten Geschwindigkeit verändem, wobei die Rechenmittel (40) die Position des Ziels (C) zu einem bestimmten Zeitpunkt, ausgehend von der bekannten Zeit und Geschwindigkeit der Positionsveränderung, berechnen.

16. Vorrichtung nach Anspruch 15, dadurch gekennzeichnet, daß die Strahlenquelle (120) und die Empfangseinrichtung (130) auf einer gemeinsamen Halterung (170) montiert sind, die vorzugsweise die Form eines C-Arms (172) hat, dessen Enden diesseits und jenseits des Zieles (C) angeordnet sind.

17. Vorrichtung nach Anspruch 16, dadurch gekennzeichnet, daß die gemeinsame Halterung (170) um eine Drehachse (174) drehbar montiert ist, die im Wesentlichen senkrecht zur Längsachse der Vorrichtung (100) angeordnet ist, welche allgemein durch die Längsachse eines Arbeitstisches (190) für die Abstützung eines in derselben Längsachse ausgestreckt liegenden Patienten definiert ist.

18. Vorrichtung nach Anspruch 16 oder 17, dadurch gekennzeichnet, daß die Mittel zur Positionsveränderung (34, 36; 70, 72; 170) so vorgesehen sind, daß sie die Position der gemeinsamen Halterung (170) translatorisch in Bezug auf das Gestell der Vorrichtung (100) in einer im wesentlichen senkrecht zur empfindlichen aktiven Oberfläche (32, 132) der Empfangseinrichtung (30; 130) verlaufenden Richtung um den gegebenen Abstand so verändern, daß zumindest die Zone des Ziels (C) abgedeckt wird; vorzugsweise mit einer Einrichtung zur Verschiebung, die einen Schlitten (176) aufweist, der auf mindestens einer oder vorzugsweise zwei Führungsschienen (178) verschiebbar montiert ist, die mit dem Gestell der Vorrichtung fest verbunden sind.

19. Vorrichtung nach einem der Ansprüche 15 bis 18, dadurch gekennzeichnet, daß die Mittel (40) zur Bestimmung der Position der Empfangseinrichtung (30; 130) zu einem gegebenen Zeitpunkt vorgesehen sind um zu bestimmen, welches Einzelelement zu welchem Zeitpunkt das Bild des Ziels (C) empfangen hat, u. zw. für jedes von zwei Bildem, die von zwei verschiedenen Winkelpositionen (S1 und S2) der Strahlenquelle (22) herrühren, sowie dazu, die genaue Lagebestimmung des Ziels (C) im Raum, auf der Grundlage der Kenntnis dieser genauen Position der beiden Einzelelemente (e_{c1} und e_{c2}), die die beiden Bilder des Ziels C ausgehend von den zwei Positionen der Strahlenquelle empfangen haben, vorzunehmen.

20. Vorrichtung nach einem der Ansprüche 15 bis 19, dadurch gekennzeichnet, daß ein Kontrollschirm (50) vorgesehen ist, auf den mindestens ein Bild der Zone des Ziels (C), das man bei der Positionsveränderung der Empfangseinrichtung (30) erhält, projiziert wird, sowie Mittel (52) zur Anzeige der Position des Ziels C auf dem Schirm und Rechenmittel (40), mit denen die Position des Ziels C mit den Anzeigemitteln (52) für jedes Bild errechnet werden kann.

21. Vorrichtung nach einem der Ansprüche 15 bis 20, dadurch gekennzeichnet, daß eine Kollimatoreinrichtung (60; 160) vorgesehen ist, die die Beschränkung der Zone der Strahlen (R1) im Raum erlaubt und somit die Strahlenbelastung des Patienten reduziert.

22. Vorrichtung nach einem der Ansprüche 17 bis 21, dadurch gekennzeichnet, daß die Strahlenquelle (180) ein Strahlenbündel in einer Anfangsrichtung (182) abgibt, die im Wesentlichen senkrecht zu einer Empfangsebene (P) liegt, die durch die genannte lineare aktive Anordnung (32; 132) und die Drehachse (174) definiert ist, wobei eine Reflexionseinrichtung (184) das Strahlenbündel zur Empfangsebene (P) zurücksendet und eine Kollimatoreinrichtung (160) mit einem Spalt (162), der parallell zur Empfangsebene (P) und in einer Richtung, die senkrecht zur Anfangsrichtung des Strahlenbündels liegt, angeordnet ist.

23. Verwendung des Verfahrens nach einem der Ansprüche 1 bis 13 und/oder der Vorrichtung nach einem der Ansprüche 14 bis 22 im Rahmen eines Geräts (10; 100), mit dem ein Ziel (C), vorzugweise durch Stoßwellen, behandelt wird.

24. Verwendung nach Anspruch 23, dadurch gekennzeichnet, daß das Behandlungsgerät ein Gerät ist, mit dem ein Ziel (C) behandelt wird, das aus der Gruppe der Steinleiden, z. B. Nieren- oder Gallensteine, der Gewebe, z. B. gutartige oder bösartige Tumoren, oder Knochen, z. B. Brüche oder osteoporotische Bereiche, gewählt wird.

25. Verwendung nach Anspruch 23 oder 24, dadurch gekennzeichnet, daß das Behandlungsgerät eine Behandlungseinrichtung (12; 112) mit einem Reflektor (14) in der Form eines Ellipsoidstumpfes, der mit einer Kopplungsflüssigkeit (16) gefüllt ist und einen inneren Brennpunkt (F1) innerhalb dieser Flüssigkeit und einen äußeren Brennpunkt (F2) aufweist, der mit dem zu behandelnden Ziel (C) in Übereinstimmung gebracht werden muß, sowie mindestens zwei symmetrisch zum inneren Brennpunkt angeordnete Elektroden (13, 15) aufweist, die durch elektrische Entladung in der Kopplungsflüssigkeit (16) die vorher genannten Stoßwellen im inneren Brennpunkt (F1) erzeugen.

## Claims

1. A method for determining the exact position of a target (C) relative to a reference point (O) determined for example by a device (12, 112) for the treatment of the target (C), comprising using an emission device (20, 120) comprising a radiation source (22, 122) emitting an image-forming radiation at least of the target (C) capable of being received by a receiver device (30, 130) of this radiation, said source (22, 122) and said receiver device (30, 130) being disposed on opposite sides of said target (C), said source (22, 122) being of a known position relative to the reference point (O), and in that the position of the target (C) is determined from at least one image of said target (C), characterized in that:
- the receiver device (30, 130) is provided in the form of a bar (32, 132) comprising a linear active part formed from a multiplicity of discrete radiation-sensitive elements (e₁ to eₙ) whose positions in space are known,
- at least one displacement of the radiation source (22, 122) and of the receiver device (30, 130) is simultaneously performed over a known distance so as to enable the emission of a radiation covering at least the zone of the target (C) according to a displacement selected from a rotation about an axis that is non perpendicular to the line defined by said linear active part, and a translation, thus obtaining at least an image of the target (C), and
- the position of the target (C) is determined from said image and said known distance of displacement.

2. The method according to claim 1, characterized in that the aforesaid displacement in translation is performed for at least two different orientations or inclinations of the associated radiation source and receiver device.

3. The method according to claim 1 or 2, characterized in that the radiation source (22, 122) and the receiver device (30, 130) are displaced simultaneously in rotation according to a rotation axis substantially parallel to the line defined by the aforesaid linear active part.

4. The method according to one of claims 1 to 3, characterized in that the radiation source (120) and the receiver device (130) are mounted onto a single common support device (170), preferably in the form of a C arm (172) having one end (172a) disposed above the target and another end (172b) disposed below the target.

5. The method according to claim 4, characterized in that the common support device (170) is mounted in rotation about a rotation axis (174) advantageously disposed substantially perpendicular to the longitudinal axis of the apparatus which is usually defined by the longitudinal axis of a so-called working table (190), supporting a patient lying on said table according to the same longitudinal axis; said rotation axis (174) being preferably mounted parallel to the sensitive active surface (132) of the receiver device (130).

6. The method according to claim 5, characterized in that the common support device (170) is displaced in translation according to a direction substantially perpendicular to the rotation axis (174) which is itself mounted parallel to the sensitive active surface (132) of the receiver device (130).

7. The method according to claim 5 or 6, characterized in that the common support device (170) is displaced in translation over a distance enabling to emit a radiation covering at least the zone of the target (C); this displacement in translation being performed for at least two different orientations or inclinations of the common support device (170) obtained by easy rotation about the rotation axis (174).

8. The method according to one of claims 1 to 7, characterized in that the image-forming radiation source is an X-ray radiation source and the aforesaid receiver device (30, 130) comprises discrete elements (e₁ to eₙ) sensitive to X-rays.

9. The method according to one of claims 1 to 7, characterized in that the radiation source is an X-ray radiation source and the aforesaid receiver device (30, 130) comprises at least a fluorescent screen that is sensitive to X-rays and discrete elements (e₁ to eₙ) that are sensitive to the fluorescent radiation from said screen, for example sensitive discrete elements that are sensitive to light photons.

10. The method according to claim 8 or 9, characterized in that the discrete elements (e₁ to eₙ) comprise components under the solid state and sensitive to X-rays, for example having semi-conductor elements.

11. The method according to one of claims 1 to 10, characterized in that the displacement of the receiver device is performed according to a predetermined known displacing speed; in that the discrete element which has received the image of the target (C) is determined for each one of the two images resulting from two different angular positions (S₁, S₂) of the radiation source (22), and that from the knowledge of the exact position of the two discrete elements (e_{C1}, e_{C2}, respectively), having received the two images of the target (C) resulting from the two positions (S₁, S₂) of the radiation source, the exact position in the space of the target (C) is determined.

12. The method according to one of the preceding claims, characterized in that a monitor screen (50) is provided on which is projected at least one image of the zone of the target (C) obtained when displacing the aforesaid receiver device (30), as well as pointing means (52) for pointing the position of the target (C) on the screen (50) and calculating means (40) for calculating the position of the target (C), notably with the pointing means (52), for each image.

13. The method according to one of the preceding claims, characterized in that a collimation device (60, 160) is provided which enables to limit in space the radiation zone (R₁), which in turn, permits to limit the irradiation of the patient; which is preferably displaced simultaneously with the aforesaid radiation source and receiver device.

14. An apparatus for determining the exact position of a target (C) relative to a reference point (O) determined for instance by a treatment device (12, 112) of the target (C), comprising an emitting device (20, 120) comprising a radiation source (22, 122) emitting an image-forming radiation at least of the target (C) capable of being received by a receiver device (30, 130) of such radiation, said source (22, 122) and said receiver device (30, 130) being disposed on opposite sides of said target (C), said source being of a known position relative to said reference point (O); and by determining means (40) for determining the position of the target (C) from at least one image of said target, characterized in that the receiver device (30, 130) is under the form of a bar (32, 132) comprising a linear active surface formed from a multiplicity of aligned discrete elements (e₁ to eₙ) sensitive to the radiation whose positions in space are known; and displacing means (34, 36; 70, 72; 170) for simultaneously displacing the radiation source (22, 122) and the receiver device (30, 130), over a known displacing distance enabling the emission of a radiation covering at least the zone of the target (C) to obtain at least an image of said target (C); said displacing means being provided for performing a displacement that is selected from a displacement in rotation about an axis that is non perpendicular to the line defined by said linear active part and a translation; said means (40) for determining the position of the target (C) comprising means for calculating the position of the target (C) from said image of the target (C) and the known distance of displacement.

15. The apparatus according to claim 14, characterized in that the displacing means (34, 36; 70, 72; 170) are provided for simultaneously displacing the radiation source (22, 122) and the receiver device (30, 130) according to a predetermined known displacing speed, said calculating means (40) for calculating the position of the target (C) at a given instant from the known displacing time and displacing speed.

16. The apparatus according to claim 15, characterized in that the radiation source (120) and the receiver device (130) are mounted on a common support device (170), preferably having a C arm shape (172) whose ends are disposed on opposite sides of the target (C).

17. The apparatus according to claim 16, characterized in that the common support device (170) is mounted in rotation about a rotation axis (174) disposed substantially perpendicular to said longitudinal axis of the apparatus (100) generally defined by the longitudinal axis of a so-called working table (190), for supporting a patient disposed according to the same longitudinal axis.

18. The apparatus according to claim 16 or 17, characterized in that the displacing means (34, 36; 70, 72; 170) are provided for displacing the common support device (170) in translation relative to the frame of the apparatus (100) in a direction substantially perpendicular to the sensitive active surface (32, 132) of the receiving device (30, 130) over said distance covering at least the zone of the target (C); preferably comprising a translation device which comprises a carriage (176) mounted in translation on at least one rail, or preferably two rails (178), for guiding in translation rigidly locked to the frame of the apparatus.

19. The apparatus according to one of claims 15 to 18, characterized in that the determining means (40) for determining the position of the receiver device (30, 130) at a given instant are provided for determining which is the discrete element (e_{C1}, e_{C2}) which has received the image of the target (C) for each one of the two images resulting from the two different angular positions (S₁, S₂) of the radiation source (22), as well as for determining the exact position in the space of the target (C) from the knowledge of this exact position of the two discrete elements (e_{C1}, e_{C2}), having received the two images of the target (C) resulting from the two positions of the radiation source.

20. The apparatus according to one of claims 15 to 19, characterized in that a monitor screen (50) is provided on which at least one image of the target zone (C) obtained when displacing the receiver device (30) is projected, as well as pointing means (52) for pointing the position of target (C) on the screen and calculating means (40) for calculating the position of the target (C) with the pointing means (52), for each image.

21. A device according to one of claims 15 to 20, characterized in that a collimation device (60, 160) is provided, enabling to limit in space the radiation zone (R₁), thereby limiting the irradiation of the patient.

22. An apparatus according to one of claims 17 to 21, characterized in that the radiation source (180) emits a radiation in an initial direction (182) substantially perpendicular to a reception plane (P) defined by the aforesaid linear active part (32, 132) and the rotation axis (174), a reflecting device (184) permitting to perform a re-emission thereof in the plane (P) of reception, as well as a collimation device (160) comprising a slot (162) disposed parallel to the reception plane (P) and in a direction perpendicular to the initial direction of said radiation.

23. A use of the method according to one of claims 1 to 13 and/or of the apparatus according to one of claims 14 to 22, within the context of an apparatus (10, 100) for the treatment of a target (C) preferably by pressure waves.

24. The use according to claim 23, characterized in that the apparatus for the treatment is an apparatus for the treatment of a target (C) selected from the group consisting of lithiasis, for example a renal or biliary lithiasis; of tissues, for example benign or malignant tumours; bones, for example a fracture or an osteoporosis zone.

25. The use according to claim 23 or 24, characterized in that the apparatus for treatment comprises a treatment device (12, 112) comprising a truncated ellipsoidal reflector (14) filled with a coupling liquid (16) comprising an intemal focus (F₁) immersed in said liquid, and an external focus (F₂) aimed to be put into coincidence with the target (C) to be treated, as well as at least two electrodes (13, 15) disposed symmetrically on opposite sides of the internal focus (F₁) for generating said pressure waves at the internal focus (F₁) through electrical discharge in the coupling liquid (16).
